Europäisches Patentamt

European Patent Office

Office européen des brevets

(19)

(11) Veröffentlichungsnummer: **0 230 625**
**B1**

(12) EUROPÄISCHE PATENTSCHRIFT

(45) Veröffentlichungstag der Patentschrift:
01.03.89

(51) Int. Cl.⁴: **C07C 37/60**

(21) Anmeldenummer: **86117703.8**

(22) Anmeldetag: **18.12.86**

(54) Verfahren zur Herstellung von Brenzkatechin und Hydrochinon.

(30) Priorität: **25.01.86 DE 3602180**

(43) Veröffentlichungstag der Anmeldung:
**05.08.87 Patentblatt 87/32**

(45) Bekanntmachung des Hinweises auf die Patenterteilung:
**01.03.89 Patentblatt 89/9**

(84) Benannte Vertragsstaaten:
**AT CH DE ES FR GB IT LI LU NL SE**

(56) Entgegenhaltungen:
**DE-A- 2 348 957**
**US-A- 3 953 530**

(73) Patentinhaber: **Degussa Aktiengesellschaft,
Weissfrauenstrasse 9, D-6000 Frankfurt am Main 1(DE)**

(72) Erfinder: **Drauz, Karl-Heinz, Dr., Flurstrasse 55,
D-6384 Freigericht 1(DE)**
Erfinder: **Prescher, Günter, Dr., Liesingstrasse 2,
D-6450 Hanau 9(DE)**
Erfinder: **Kleemann, Axel, Dr., Bornweg 36,
D-6052 Mühlheim 2(DE)**
Erfinder: **Ritter, Gebhard, Dr., Fürstenbergstrasse 2,
D-6450 Hanau 9(DE)**

## Beschreibung

Die Erfindung betrifft die Herstellung von Brenzkatechin und Hydrochinon durch Hydroxylieren von Phenol mit wäßrigem Wasserstoffperoxid.

Brenzkatechin und Hydrochinon werden bei der Herstellung von Farbstoffen in der Kunststoffproduktion sowie in der Foto- und Pflanzenschutzmittel-Industrie eingesetzt.

Es ist nach dem Verfahren der DE-PS 2 064 497 bekannt, die Kernhydroxylierung aromatischer Verbindungen, vor allem von Phenol, mit Wasserstoffperoxid in Gegenwart einer starken Säure durchzuführen. Das Reaktionsmedium soll dabei zu Anfang nicht mehr als 20 Gew.-% Wasser, bevorzugt unter 10 Gew.-% Wasser, enthalten.

Der pH-H$_2$O-Wert der starken Säuren wird unter –0,1, vorzugsweise unter –1, angegeben. Schwefelsäure und Perchlorsäure scheinen bevorzugt zu sein.

Dieses Verfahren wird aber von der Anmelderin der DE-PS 2 064 497 selbst in ihrer späteren Patentschrift DE-PS 2 658 545 recht negativ beurteilt. So sollen zwar die Ausbeuten an Hydroxylierungsprodukten bei gleichzeitiger Verwendung der starken Säuren und von Komplexbildnern für Metalle wie Pyrophosphorsäure "ausgezeichnet" sein, der Umwandlungsgrad der aromatischen Verbindung aber unter 30% liegen. In der Praxis würden sogar Werte von 4 bis 10% Umwandlungsgrad nicht überschritten.

Dies bedeute eine Begrenzung der Produktivität der Apparatur und die Rückführung eines bedeutenden Volumens an Ausgangsstoff.

Daher wäre eine möglichst hohe Reaktionsgeschwindigkeit wichtig. Diese hinge – bei gegebener Temperatur und Wassermenge – von Art und Menge der eingesetzten Säure ab. Unabhängig von der Art der Säure wäre es aber wünschenswert, die Reaktionsgeschwindigkeit zu erhöhen, und zwar ohne Erhöhung der Säuremenge, da letztere durch Auswaschen verloren ginge und außerdem auch die Korrosion durch die starke Säure nicht außer acht gelassen werden könne.

So wird in der DE-PS 2 658 545 als Verbesserung des obengenannten Verfahrens noch neben den dort genannten Katalysatoren und Stabilisatoren die zusätzliche Verwendung von aromatischen Aldehyden, wie Benzaldehyd, vorgeschlagen. Die Hydroxylierung von Phenol und substituierten Phenolen mit Wasserstoffperoxid findet also in Gegenwart starker Säuren, von Metallkomplexbildnern und aromatischen Aldehyden statt.

Anstelle von zwei, die Reaktion beeinflussenden Komponenten, werden nun also drei eingesetzt. Diese Komponenten müssen nicht nur aus dem Reaktionsgemisch abgetrennt werden und sind – als nicht rückgewinnbar – verloren, sondern die Komponente "aromatischer Aldehyd" unterliegt auch einer Oxydation mit Wasserstoffperoxid, was die Gefahr einer Verunreinigung des Endproduktes einschließt.

Höhere Ausbeuten von etwa 70–76% – und in zwei Fällen über 80% – werden aber auch hier nur dann erhalten, wenn Phenol/Wasserstoffperoxidverhältnisse von 20:1 und Wasserstoffperoxid von etwa 85% eingesetzt werden. In diesen Fällen muß aber, wie in der DE-PS 2 064 497, "ein bedeutendes Volumen an Ausgangsstoff rückgeführt" werden, was entsprechende technische Anlagen benötigt. Auch die Reaktoren selbst müssen dementsprechend groß ausgelegt werden.

Da beim Verfahren der DE-PS 2 064 497 die Ausbeute an Brenzkatechin und Hydrochinon bei Verringerung des Verhältnisses von Phenol zu Wasserstoffperoxid erheblich sinkt – bei einem Verhältnis von 10:1 liegt sie bei 60%, bei einem solchen von 5:1 bei 47%, siehe Beispiel 7 – scheint die Anwesenheit eines großen Überschusses an Phenol bei der Durchführung der Hydroxylierung mit starken Säuren eine Notwendigkeit zu sein, ganz abgesehen davon, daß die in der DE-PS 2 064 497 genannten starken Säuren, wie Schwefelsäure, Perchlorsäure als Katalysatoren nur eine unzureichende Wirkung hatten.

Man hatte deshalb schon angenommen, durch Verwendung von wasserfreien Lösungen von Wasserstoffperoxid die katalytische Wirkung der starken Säuren zu verbessern, siehe DE-PS 2 410 742 und DE-OS 2 410 758; jedoch wurde auch hier die Anwesenheit von Phosphorverbindungen als Komplexbildner für wesentlich gehalten. Außerdem wurde darauf hingewiesen, daß die Reaktion am schnellsten bei hohen Säurekonzentrationen verlaufe. Die Frage der Korrosion durch starke Säuren war also auch hier nicht gelöst.

Einen wesentlichen Fortschritt gegenüber den genannten Verfahren brachten die Verfahren der DE-PS 3 308 737, DE-PS 3 308 769, DE-PS 3 308 763 und DE-PS 3 308 726, bei denen die Hydroxylierung von Phenol oder seinen Derivaten durch organische Lösungen von Wasserstoffperoxid in Gegenwart von Schwefeldioxid oder Selendioxid durchgeführt wurde.

Bei diesen Verfahren traten die störenden Folgen der starken Säuren, wie Korrosion, nicht ein, noch war es nötig, zur Erhöhung der Aktivität zusätzliche Verbindungen, wie Aldehyde, oder auch Komplexbildner, wie Phosphorderivate, einzusetzen.

Obwohl die Katalysatoren in sehr geringen Mengen eingesetzt wurden, war die Reaktionsgeschwindigkeit groß; es wurden sehr günstige Raum-Zeitausbeuten und sehr gute Ausbeuten erhalten. Durch ihre sehr geringe Menge beanspruchen Schwefeldioxid und Selendioxid auch keine speziellen Abtrennmethoden.

Nach dem Stand der Technik schien zunächst einmal nur die Verwendung von wasserfreien Lösungen von Wasserstoffperoxid zu besseren Umsätzen und Ausbeuten zu führen. Bei Verwendung von starken Säuren als Katalysator bestand aber nach wie vor die Schwierigkeit von deren Abtrennung sowie das Auftreten von Korrosion.

Hier brachte der Einsatz von Schwefeldioxid oder Selendioxid einen wesentlichen Fortschritt. Nur beanspruchten auch diese Verfahren wasserfreie Lösungen von Wasserstoffperoxid, die nicht mehr als 1 Gew.-%, bevorzugt unter 0,5 Gew.-%

Wasser, enthalten sollten. Diese Arbeitsweise erfordert auch zusätzliche Anlagen zur Abtrennung und Rückführung des organischen Lösungsmittels.

Das Verfahren gemäß der DE-OS 2 348 957 richtet sich auf die Kernhydroxylierung aromatischer Verbindungen, darunter Phenol, mit einer wäßrigen Wasserstoffperoxidlösung, wobei man in Anwesenheit eines Metalloids aus der Gruppe von Schwefel, Selen, Tellur, Phosphor, Arsen und Antimon arbeitet. Dieses Verfahren wurde ebenso wie dasjenige mit Schwefelsäure als Katalysator nachgearbeitet, wobei mit Schwefel als Katalysator die Kernhydroxylierung wesentlich langsamer erfolgt und zu geringeren Ausbeuten führt als mit der etwa äquivalenten Menge Schwefelsäure als Katalysator – vgl. nachstehende Beispiele 6 und 7.

Aufgabe der vorliegenden Erfindung ist es nun, die Kernhydroxylierung von Phenol mit Schwefeldioxid oder Selendioxid als Katalysator in technisch einfacher Form, aber mit sehr guten Ausbeuten und Selektivitäten durchzuführen.

Gelöst wird diese Aufgabe durch ein Verfahren zur Herstellung von Brenzkatechin und Hydrochinon durch Kernhydroxylierung von Phenol mit Wasserstoffperoxid bei 40 bis 160°C und einem Molverhältnis von Phenol zu Wasserstoffperoxid von 5 bis 20:1 in Gegenwart von 0,0001 bis 0,01 Mol Schwefeldioxid oder Selendioxid pro Mol Wasserstoffperoxid, das dadurch gekennzeichnet ist, daß man 30 bis 85 gew.-%iges wäßriges Wasserstoffperoxid einsetzt.

Das Molverhältnis von Phenol zu Wasserstoffperoxid liegt bevorzugt bei 7 bis 15:1, ganz bevorzugt bei 10:1.

Wasserstoffperoxid wird in handelsüblichen Lösungen in den entsprechenden Konzentrationen eingesetzt; bevorzugt wird 70 bis 85 gew.-%iges wäßriges Wasserstoffperoxid verwendet.

Schwefeldioxid wird flüssig oder gasförmig aus handelsüblichen Stahlflaschen oder als Lösungen in inerten Lösungsmitteln, wie z.B. Phenol oder Alkylestern, verwendet. SeO$_2$ wird in p.a. Form eingesetzt.

Die Umsetzungen werden bei Temperaturen von 40–160°C durchgeführt. Der Druck ist nicht entscheidend; im allgemeinen wird bei Normaldruck gearbeitet.

Das Verfahren kann sowohl diskontinuierlich als auch kontinuierlich durchgeführt werden. Das bei dem erfindungsgemäßen Verfahren rezyklierte Phenol bedarf keiner besonderen Behandlung. Vorzugsweise wird das durch die Umsetzung verbrauchte Phenol vor Abdestillieren des überschüssigen Phenols zugesetzt.

Der technische Fortschritt des erfindungsgemäßen Verfahrens liegt einmal in seiner einfachen technischen Durchführung: anstelle von organischen Wasserstoffperoxid-Lösungen kann direkt wäßriges Wasserstoffperoxid eingesetzt werden. Ferner werden durch Verwendung von Schwefeldioxid oder Selendioxid in äußerst geringen Mengen die Schwierigkeiten vermieden, die durch Verwendung von starken Säuren auftreten, wie aufwendiges Abtrennen dieser Katalysatoren und Auftreten von Korrosionen. Auch fällt das Aufsalzen der Abwässer durch Neutralisation der Säuren fort.

Es kommt hinzu, daß Aktivatoren für eine ausreichende Reaktionsgeschwindigkeit bei Schwefeldioxid und Selendioxid nicht nötig sind, ebenso nicht der Zusatz von Stabilisatoren. Damit werden zusätzliche technische Einrichtungen und bei der Sauerstoffempfindlichkeit gerade der wirkungsvollen Aktivatoren auch die Verschmutzung des Endproduktes durch Oxydationsprodukte der Aktivatoren vermieden.

Ferner führt die hohe Reaktionsgeschwindigkeit zu einer erhöhten Raum-Zeitausbeute.

Zu der vereinfachten technischen Durchführung kommt aber hinzu, daß der Umsatz an Wasserstoffperoxid hoch und die Ausbeute an Brenzkatechin und Hydrochinon mindestens so hoch wie bei den Verfahren des Standes der Technik ist. Da sich das nicht umgesetzte Wasserstoffperoxid zersetzt und nicht zu Nebenprodukten geführt hat, ist auch die Selektivität bei dem erfindungsgemäßen Verfahren hoch.

Diese Ausbeuten sind auch bei einem gegenüber dem Stand der Technik verringerten Verhältnis von Phenol zu Wasserstoffperoxid erhältlich, siehe Beispiele 1–3 und 4. Die Abhängigkeit der Ausbeuten von der Größe des Phenolüberschusses ist damit nicht so groß wie bei den bekannten, mit wäßrigem Wasserstoffperoxid arbeitenden Verfahren.

Die Erfindung wird durch die nachstehenden Beispiele weiter erläutert:
Das verwendete Schwefeldioxid ist 99,75 gew.-%ig.
Die Ergebnisse wurden mit Hochdruckflüssigkeitschromatografie (HPLC) und Gas-Flüssigkeitschromatografie (GLC) bestimmt.

Es wurde unter Stickstoff gearbeitet, obwohl auch bei Anwesenheit von Luft keine Änderungen an den Ergebnissen beobachtet wurden.

Beispiel 1

94 g (1,0 Mol) destilliertes Phenol werden in einem Vielhalskolben unter N$_2$-Atmosphäre auf 50°C erwärmt. Zu dieser gerührten Mischung gibt man stufenweise 0,5 g einer 2,5 gewichtsprozentigen Lösung von Schwefeldioxyd in Phenol und 2 g (0,05 Mol) 85 gewichtsprozentiges Wasserstoffperoxid. Die Temperatur erhöht sich danach auf 87°C.

Nach 5 Minuten wird bereits ein Wasserstoffperoxidumsatz von 98% bestimmt und nach 10 Minuten ist das H$_2$O$_2$ vollständig umgesetzt.

Das Reaktionsgemisch enthält dann 3,0 g Brenzkatechin und 1,65 g Hydrochinon, was einer Gesamtausbeute an Dihydroxybenzolen von 84,5%, bezogen auf eingesetztes Wasserstoffperoxid, entspricht.

Beispiel 2

94 g (1,0 Mol) destilliertes Phenol werden unter N$_2$-Atmosphäre auf 110°C erwärmt. Zu dieser gerührten Mischung gibt man stufenweise 2,5 g (0,05 Mol) 70%iges Wasserstoffperoxid und 0,5 g einer 2,7 gewichtsprozentigen Lösung von Schwefeldioxid in Phenol. Die Temperatur in der Reaktionslösung erhöht sich danach auf 143°C.

Nach Abklingen der Exotherme wird nach 10 Minuten ein Wasserstoffperoxidumsatz von 99,6% bestimmt.

Das Reaktionsgemisch enthält dann 1,55 g Hydrochinon und 3,1 g Brenzkatechin, was einer Gesamtausbeute von 82%, bezogen auf eingesetztes Wasserstoffperoxid, entspricht.

Beispiel 3

94 g (1,0 Mol) destilliertes Phenol werden unter N2-Atmosphäre auf 110°C erwärmt und stufenweise mit 3,5 g (0,05 Mol) 50%igem H2O2 und 0,5 g einer 3,0 gewichtsprozentigen Lösung von Schwefeldioxid in Phenol versetzt. Die Temperatur in der Reaktionslösung erhöhte sich danach auf 118°C.

Nach Abklingen der Exotherme wird nach 10, 20 und 40 Minuten ein Wasserstoffperoxidumsatz von 80, 92 und 99,8% bestimmt.

Das Reaktionsgemisch enthält dann 3,16 g Brenzkatechin und 1,45 g Hydrochinon, was einer Gesamtausbeute an Dihydroxybenzolen von 83,5%, bezogen auf eingesetztes Wasserstoffperoxid, entspricht.

Beispiel 4

47 g (0,5 Mol) destilliertes Phenol werden in einem Vielhalskolben unter N2-Atmosphäre auf 110°C erwärmt. Zu dieser Lösung gibt man 0,5 g einer 2,7 gewichtsprozentigen Lösung von Schwefeldioxid in Phenol und 2,44 g (0,05 Mol) 70%iges Wasserstoffperoxid. Die Temperatur in der Reaktionslösung erhöht sich danach auf 146°C. Nach Abklingen der Exotherme werden nach bestimmten Zeitabschnitten folgende H2O2-Umsätze bestimmt:

|  | H2O2-Umsatz | H2O2-Ausbeute |
| --- | --- | --- |
| 2 Minuten = | 93,5% | 78,5% |
| 5 Minuten = | 96,8% | 79,8% |
| 10 Minuten = | 99,2% | 80,7% |

Das Reaktionsgemisch enthält dann nach 10 Minuten 1,25 g Hydrochinon und 3,19 g Brenzkatechin, was einer Gesamtausbeute von 80,7%, bezogen auf eingesetztes Wasserstoffperoxid, entspricht.

Es wurden 43 g Phenol wiedergefunden; das entspricht einer Phenolselektivität von 94%.

Beispiel 5

47 g (0,5 Mol) destilliertes Phenol werden in einem Vielhalskolben unter N2-Atmosphäre auf 110°C erwärmt. Zu dieser Lösung gibt man 0,5 g einer 2,5%igen Lösung von Schwefeldioxid in n-Propylacetat und 4,86 g (0,1 Mol) 70%iges Wasserstoffperoxid.

Die Temperatur in der Reaktionslösung erhöht sich danach auf 133°C.

Nach 2, 5 und 10 Minuten wurden folgende H2O2-Umsätze gemessen:

2 Minuten = 83%
5 Minuten = 98%
10 Minuten = 99,3%.

Das Reaktionsgemisch enthält nach 10 Minuten 1,96 g Hydrochinon und 4,96 g Brenzkatechin, was einer Gesamtausbeute von 62,9%, bezogen auf eingesetztes Wasserstoffperoxid, entspricht.

Beispiel 6 (Vergleichsbeispiel)

47 g (0,5 Mol) destilliertes Phenol werden in einem Vielhalskolben unter N2-Atmosphäre auf 110°C erwärmt. Zu dieser Mischung gibt man 8 mg (0,25 mmol) Schwefel und setzt nach vollständiger Auflösung des Schwefels 2,43 g (0,05 Mol) 70%iges Wasserstoffperoxid hinzu. Die Reaktionstemperatur in der Lösung blieb unverändert bei 110°C stehen. Man bestimmte in folgenden Zeitabständen folgende H2O2-Umsätze:

|  | H2O2-Umsatz | H2O2-Ausbeute |
| --- | --- | --- |
| 2 Minuten = | 8% | 0% |
| 15 Minuten = | 20,4% | 10,8% |
| 30 Minuten = | 25% | 12,0% |

Dieses Vergleichsbeispiel zu Beispiel 4 zeigt, daß SO2 bei äquimolarem Katalysatoreinsatz den wesentlich besseren Katalysator darstellt. Die angegebenen Wasserstoffperoxidausbeuten beziehen sich auf eingesetztes Wasserstoffperoxid.

Beispiel 7 (Vergleichsbeispiel)

47 g (0,5 Mol) destilliertes Phenol werden in einem Vielhalskolben unter N2-Atmosphäre auf 110°C erwärmt. Zu dieser Lösung gibt man 25 mg (0,24 mmol) 96–98%ige Schwefelsäure und 2,43 g (0,05 Mol) 70%ige Wasserstoffperoxidlösung.

Die Temperatur in der Lösung erhöht sich danach auf 136°C.

Nach 2, 5 und 10 Minuten bestimmte man folgende H2O2-Umsätze:

|  | H2O2-Umsatz | H2O2-Ausbeute |
| --- | --- | --- |
| 2 Minuten = | 64% | 50,1% |
| 5 Minuten = | 94% | 72% |
| 10 Minuten = | 98,9% | 74% |

Die angegebenen Dihydroxybenzol-Ausbeuten sind auf eingesetztes Wasserstoffperoxid bezogen. Dieses Vergleichsbeispiel zu Beispiel 4 zeigt, daß SO2 bei äquimolarem Katalysatoreinsatz den wesentlich besseren Katalysator darstellt.

Beispiel 8 (kontinuierliche Fahrweise)

In ein senkrecht stehendes Strömungsrohr aus Glas bringt man pro Stunde 135 g (85%iges) Wasserstoffperoxid gelöst in 3540 g Phenol ein. Zu dieser Mischung dosiert man die gewünschte Menge gasförmiges SO2 über eine Düse hinzu. Die SO2-

Konzentration dieser Mischung beträgt 0,01 Mol, bezogen auf 1 Mol eingesetztes Wasserstoffperoxid. Dieses Reaktionsgemisch wurde vor Erreichen des Strömungsrohres über einen Wärmeaustauscher auf die gewünschte Reaktionstemperatur von 140°C erwärmt. Nach 60 Sekunden Verweilzeit bestimmte man am Ende des Strömungsrohres den $H_2O_2$-Umsatz zu 98,5% bei einer Wasserstoffperoxid-Ausbeute an Dihydroxybenzolen von 86,3%. Der Quotient Brenzkatechin zu Hydrochinon wurde mit 1,8 bestimmt.

## Patentansprüche

1. Verfahren zur Herstellung von Brenzkatechin und Hydrochinon durch Kernhydroxylierung von Phenol mit Wasserstoffperoxid bei 40 bis 160°C und einem Molverhältnis von Phenol zu Wasserstoffperoxid von 5 bis 20:1 in Gegenwart von 0,0001 bis 0,01 Mol Schwefeldioxid oder Selendioxid pro Mol Wasserstoffperoxid, dadurch gekennzeichnet, daß man 30 bis 85 gew.-%iges wäßriges Wasserstoffperoxid einsetzt.

2. Verfahren nach Anspruch 1, dadurch gekennzeichnet, daß man wäßriges Wasserstoffperoxid in einer Konzentration von 70 bis 85 Gew.-% einsetzt.

3. Verfahren nach Anspruch 1 oder 2, dadurch gekennzeichnet, daß das Molverhältnis von Phenol zu Wasserstoffperoxid bei 7 bis 15:1, bevorzugt bei 10:1, liegt.

## Claims

1. Process for the preparation of catechol and hydroquinone by ring hydroxylation of phenol with hydrogen peroxide at 40 to 160°C and a molar ratio of phenol to hydrogen peroxide of 5 to 20:1 in the presence of 0.0001 to 0.01 mole of sulphur dioxide or selenium dioxide per mole of hydrogen peroxide, characterized in that 30 to 85% strength by weight aqueous hydrogen peroxide is used.

2. Process according to Claim 1, characterized in that the aqueous hydrogen peroxide is used in a concentration of 70 to 85% by weight.

3. Process according to Claim 1 or 2, characterized in that the molar ratio of phenol to hydrogen peroxide is 7 to 15:1, preferably 10:1.

## Revendications

1. Procédé d'obtention de pyrocatéchol et d'hydroquinone par hydroxylation sur le noyau du phénol avec le peroxyde d'hydrogène à une température allant de 40 à 170°C et pour un rapport molaire du phénol au peroxyde d'hydrogène de 5 à 20:1 en présence de 0,0001 à 0,01 mol d'anhydride sulfureux ou de dioxyde de sélénium par mol de peroxyde d'hydrogène, caractérisé on ce que l'on met en jeu du peroxyd d'hydrogène aqueux à 30 à 85% en poids.

2. Procédé selon la revendication 1, caractérisé en ce que l'on met en jeu le peroxyde d'hydrogène aqueux à une concentration de 70 à 85% en poids.

3. Procédé selon la revendication 1 ou 2, caractérisé en ce que le rapport molaire du phénol au peroxyde d'hydrogène se situe de 7 à 15:1, de préférence à 10:1.